(19) Europäisches Patentamt
European Patent Office
Office européen des brevets

(11) Veröffentlichungsnummer: **0 137 262**
**B1**

(12) **EUROPÄISCHE PATENTSCHRIFT**

(45) Veröffentlichungstag der Patentschrift: **15.02.89**

(21) Anmeldenummer: **84110092.8**

(22) Anmeldetag: **24.08.84**

(51) Int. Cl.⁴: **C 07 D 303/28**, C 07 D 303/18, C 07 D 303/12, C 07 C 50/16, A 61 K 31/335, A 61 K 31/12

(54) **Mono- und bisfunktionelle Anthrachinon-(oxy-2,3-oxidopropane), Verfahren zu ihrer Herstellung und ihre Verwendung als Arzneimittel.**

(30) Priorität: **31.08.83 DE 3331296**

(43) Veröffentlichungstag der Anmeldung: **17.04.85 Patentblatt 85/16**

(45) Bekanntmachung des Hinweises auf die Patenterteilung: **15.02.89 Patentblatt 89/7**

(84) Benannte Vertragsstaaten: **AT BE CH DE FR GB IT LI LU NL SE**

(56) Entgegenhaltungen:
**L.u.M. FIESER "Organische Chemie", 2. Auflage, 1982, VERLAG CHEMIE, Weinheim, pp. 1368-1379**
**CHEMICAL ABSTRACTS, vol. 90, no. 11, 12. März 1979, Columbus, Ohio, USA, R. BUDZIAREK, "The reaction of sodium sulfite and bisulfite with nitro- and hydroxylamino-anthraquinones", p. 591, abstract no. 87118z**
**CHEMICAL ABSTRACTS, vol. 90, no. 4, 12. März 1979, Columbus, Ohio, USA, K.A.BELL, I.J.FLATMAN, P.GOLDBORN, A.PACHL, F.SCHEINMANN "Elimination of an allyloxy-group during the Claisen rearrangement of 1,2-diallyloxyanthraquinone", p. 591, abstract no. 87121v**
**CHEMICAL ABSTRACTS, vol. 98, no. 9, 28. Februar 1983, Columbus, Ohio, USA, K. SEGUCHI, A. OZASA "Synthesis of aromatic ether by phase transfer-catalyzed SNAr reaction", p. 586, abstract no. 71620m**
**CHEMICAL ABSTRACTS, vol. 95, no. 4, 27. Juli 1981, Columbus, Ohio, USA, I.W. STAPLETON, P.J. WATERS "Dyeing of wool-polyester blends with reactive disperse dyes: variation of dye distribution and reactivity for wool", p. 62, abstract no. 26492y**
**CHEMICAL ABSTRACTS, vol. 93, no. 16, 20. Oktober 1980, Columbus, Ohio, USA, CHEN, MING-HO "Heat transfer printing of wool textiles", p. 64, abstract no. 151542z**

(73) Patentinhaber: **HOECHST AKTIENGESELLSCHAFT, Postfach 80 03 20, D-6230 Frankfurt am Main 80 (DE)**

(72) Erfinder: **Stache, Ulrich, Dr., Goldgrabenstrasse 20, D-6238 Hofheim am Taunus (DE)**
Erfinder: **Kraemer, Hans Peter, Dr., Birkenweg 16, D-3550 Marburg (DE)**
Erfinder: **Sedlacek, Hans- Harald, Dr., Am Sonnenhang 3, D-3550 Marburg (DE)**

(56) Entgegenhaltungen: (Fortsetzung)
**CHEMICAL ABSTRACTS, vol. 95, no. 17, 26. Oktober 1981, Columbus, Ohio, USA, R.C.CAMBIE, M.G.DUNLOP, W.I.NOALL, P.S.RUTLEDGE, P.D.WOODGATE "Experiments directed towards the synthesis of anthracyclones. VI. Claisen rearrangements of 1,4-bis(prop-2'-enyloxy)anthraquinone", p. 628, abstract no. 150252j**
**CHEMICAL ABSTRACTS, vol. 96, no. 1, 4. January 1982, Columbus, Ohio, USA, R.C.CAMBIE, Z.D.HUANG, W.I.NOALL, P.S.RUTLEDGE, P.D.WOODGATE, "Experiments directed towards the synthesis of anthracyclones. V. Double Claisen rearrangement of 1,4-bis(allyloxy)anthraquinones", p. 535, abstract no. 5890g**
**CHEMICAL ABSTRACTS, vol. 98, no. 11, 14. März 1983, Columbus, Ohio, USA, I.K.BODDY, P.J.BONIFACE, R.C.CAMBIE, P.A.CRAW, D.S.LARSEN, H.McDONALD, P.S.RUTLEDGE, P.D.WOODGATE, "Reductive Claisen reaarangements of anthraquinone allyl ethers", p. 516, abstract no. 88942r**
**CHEMICAL ABSTRACTS, vol. 97, no. 5, 2. August 1982, Columbus, Ohio, USA, S.I.POPOV, V.P.VOLOSENKO, "Reactions of 1-acetoxy-9,10-anthraquinones with formation of 1m10-anthraquinones and 10-anthrones", p. 543, abstract no. 38637d**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



LIBER, STOCKHOLM 1989

**Beschreibung**

Die vorliegende Erfindung betrifft neue mono- und bisfunktionelle Anthrachinon-(oxy-2,3-oxidopropane) der Formel I

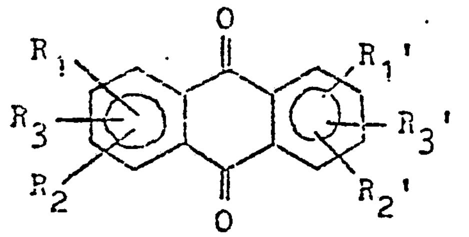

I

worin
$R_1$ und $R_1'$ gleich oder verschieden sind und Wasserstoff, Hydroxy oder den Rest

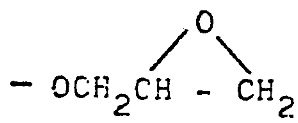

bedeuten,
$R_2$ und $R_2'$ gleich oder verschieden sind und Wasserstoff oder eine oder mehrere Hydroxy-, $C_1$-$C_4$-Alkyl-, $C_1$-$C_4$-Alkoxy-, $C_1$-$C_4$-Alkoxycarbonyl-, $C_1$-$C_4$-Alkanoxyloxy-, Halogen- oder Nitrogruppen bedeuten, und
$R_3$ und $R_3'$ verschieden sind und Wasserstoff oder den Rest

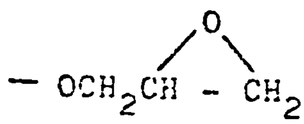

bedeuten.

Unsubstituiertes Anthrachinon und mit Nitro-, Alkoxy- oder Phenoxygruppen oder Halogen substituierte Anthrachinon-Derivate sind bereits bekannt, z. B. aus: Fieser und Fieser, Organ. Chemie, Weinheim 1982, S. 1370, S. 1375 (1982); C. A. 90, Nr. 87118z (1979); C. A. 97, Nr. 38637d (1982) und C. A. 98, Nr. 71620m (1983).

Die Erfindung beftrifft ferner ein Verfahren zur Herstellung von Verbindungen der Formel I, die dadurch gekennzeichnet sind, daß man ein Mono- oder Di- oder Polyhydroxyanthrachinon der Formel II

II

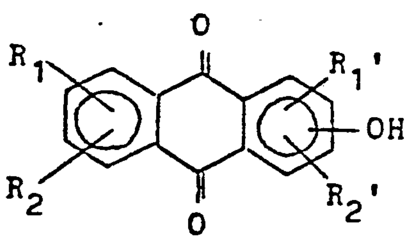

(Position 1,2,3,4,5, 6,7 oder 8)

worin
$R_1$ und $R_1'$ H und/oder OH bedeuten und
$R_2$ und $R_2'$ die oben angegebene Bedeutung haben,
entweder

1. mit Epichlor- oder Epibromhydrin in Gegenwart von Basen, gegebenenfalls unter Zusatz eines inerten organischen Lösungsmittels bei Temperaturen von 0°C bis zum Siedepunkt des jeweiligen Reaktionsgemisches zu den erfindungsgemäßen Verbindungen der Formel I

oder

2. mit Epichlor- oder Epibromhydrin in Gegenwart von Basen, gegebenenfalls unter Zusatz eines inerten organischen Lösungsmittels bei Temperaturen von 0°C bis zum Siedepunkt des jeweiligen Reaktionsgemisches zu Verbindungen der Formel III

III 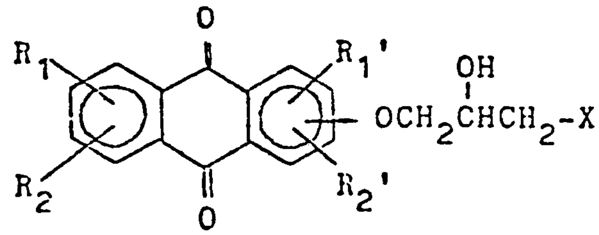 X=Hal=F,Cl,Br,J

worin

OH

$R_1$ und $R_1'$ H, OH oder $OCH_2CHCH_2Hal$ bedeuten und
$R_2$ und $R_2'$ die oben angegebene Bedeutung haben
umsetzt und diese durch Behandlung mit anorganischen oder organischen Basen, gegebenenfalls unter Zusatz eines inerten organischen Lösungsmittels und/oder Wasser unter Eliminierung von HalH zu den erfindungsgemäßen Verbindungen der Formel I umsetzt
oder
3.a) mit Glycidol, der Formel IV

IV 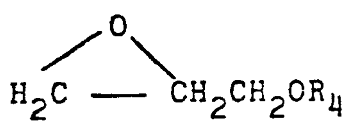

worin
$R_4$ H, Alkyl, Acyl oder einen Alkyl- oder Arylsulfonsäureester bedeuten, vorzugsweise H,
gegebenenfalls unter Zusatz einer Base, eines inerten organischen Lösungsmittels, gegebenenfalls unter Zugabe von Wasser zu Verbindungen der Formel V umsetzt

V 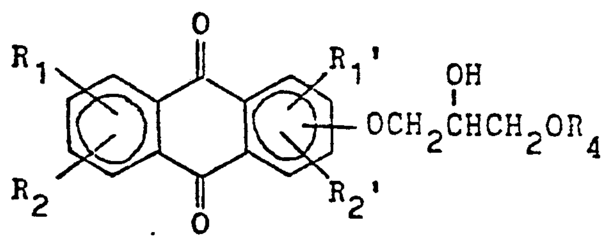

worin

OH

$R_1$ und $R_1'$ H, OH oder $OCH_2CHCH_2OR_4$
und $R_2$ und $R_2'$ sowie $R_4$ die oben angegebenen Bedeutungen haben,
und die Verbindungen der Formel V, wenn $R_4$ Alkyl oder vorzugsweise Acyl bedeuten, zu Verbindungen der allgemeinen Formel V mit der Bedeutung $R_4$ = H hydrolysiert und diese durch Umsetzung mit einem Alkyl- oder Arylsulfonsäurehalogenid zu Verbindungen der Formel V, in der $R_4$ einen Alkyl- oder Arylsulfonsäurerest bedeuten, umsetzt, und diese Verbindung, gegebenenfalls nach nucleophilem Austausch der Alkyl- oder Arylsulfonsäureestergruppe $R_4$ gegen Halogen, wie F, Cl, Br, J, durch Behandlung mit Basen, falls die Eliminierung nicht schon ohne diesen Zusatz abläuft, unter Eliminierung von Alkyl- oder Arylsulfonsäure oder Halogenwasserstoffsäure, wenn $R_4$ = F, Cl, Br, J ist, zu den erfindungsgemäßen Verbindungen der Formel I umsetzt
b) mit 1-Halogen-2,3-dihydroxypropan oder 1-Alkyl- bzw. 1-Arylsulfonato-2,3-dihydroxypropan der Formel VI

VI $R_5CH_2CHOHCH_2OH$,

worin
$R_5$ Chlor, Brom, Jod oder einen Alkyl- oder Arylsulfonsäureesterrest bedeuten,
mit Basen, vorzugsweise mit starken anorganischen Alkalien, gegebenenfalls unter Zusatz von inerten organischen Lösungsmitteln zu Verbindungen der Formel VII umsetzt

VII 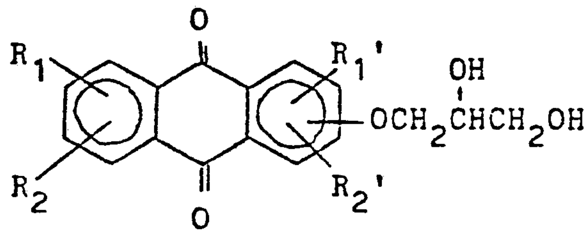

worin

$R_1$ und $R_1'$ H, OH oder $OCH_2CH(OH)CH_2OH$ bedeuten
und
$R_2$ und $R_2'$ die oben angeführte Bedeutung haben,
und diese dann gemäß 3.a) weiter zu den Verbindungen der Formel I umsetzt.
c) mit einem Derivat des 1-Halogen- bzw. 1-Alkyl- oder 1-Arylsulfonato-2,3-dihydroxypropans der Formel VIII

VIII 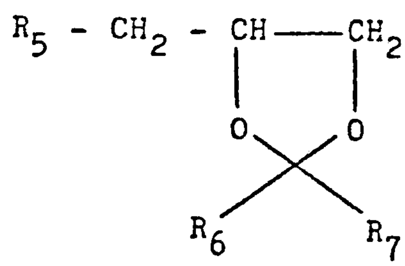

worin
$R_5$ obige Bedeutung und
$R_6$ und $R_7$ jeweils allein oder auch zusammen H, Alkyl, Phenyl, Benzyl bedeuten, mit Basen, vorzugsweise mit starken anorganischen Alkalien, gegebenenfalls unter Zusatz von inerten organischen Lösungsmitteln zu Verbindungen der Formel IX umsetzt

IX 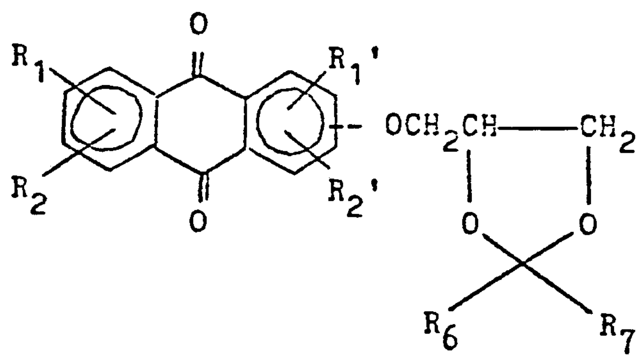

worin
$R_1$ und $R_1'$ H, OH, $OCH_2$-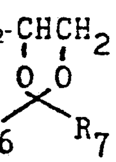 und

$R_2$, $R_6$, $R_7$ obige Bedeutungen haben,
diese dann durch saure Hydrolyse zu Verbindungen der Formel VII umsetzt und diese dann gemäß 3.a) weiter zu den erfindungsgemäßen Verbindungen der Formel umsetzt,
oder

4. mit Alkylchlorid, -bromid oder -jodid in Gegenwart von Basen, gegebenenfalls unter Zusatz eines inerten organischen Lösungsmittels bei Temperaturen von 0°C bis zum Siedepunkt des jeweiligen Reaktionsgemisches zu den Verbindungen der Formel X umsetzt

X 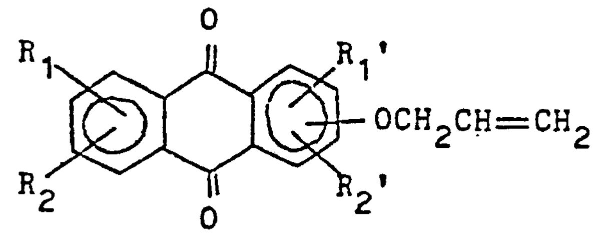

worin
$R_1$ und $R_1'$ H, OH oder $OCH_2CH=CH_2$ bedeuten und
$R_2$ und $R_2'$ obige Bedeutungen haben
und diese dann mit organischen Alkyl- oder Arylpercarbonsäuren in inerten organischen Lösungsmitteln oder mit dem ($J_2 \cdot AgO$) Komplex zu den erfindungsgemäßen Verbindungen der Formel I umsetzt.

Zur Umsetzung der Mono- oder Di- oder Polyhydroxyanthrachinone der Formel II zu mono- und bisfunktionellen Anthrachinon-(oxy-2,3-oxidopropanen) der Formel geht man wie folgt vor:

**Verfahrensvariante 1** (Formel II → Formel I)

Als Basen für die Umsetzung kommen vorzugsweise anorganische Basen, insbesondere Natrium-, Kaliumhydroxid bzw. deren Methylate, Äthylate oder tert.-Butylate, ferner Natrium-Kalium- und calciumcarbonate oder -bicarbonate in Betracht. DOWEX®-1 (OH-Form) kann anstelle von Basen benutzt werden. Als inerte Lösungsmittel dienen vorzugsweise Aceton, Äthylmethylketon, Dimethylformamid, Acetonitril, HMPT oder Alkohole, z. B. tert.-Butanol. Die Reaktion wird vorzugsweise zwischen 0°C und den Siedepunkten der verwendeten Lösungsmittel durchgeführt. Beim Verätherungsvorgang entstehendes Wasser wird vorzugsweise über ein Reverswasserabschneider ausgekreist. Das ist insbesondere dann der Fall, wenn ausschließlich in Epichlorhydrin oder Epibromhydrin ohne weiteren Lösungsmittelzusazt gearbeitet wird. Eine schonende und vorteilhafte Verfahrensvariante besteht in 1 bis 480 stündigem Rückflußkochen in Gegenwart der halogenwasserstoff-bindenden Kaliumcarbonate in Äthylmethylketon. Bei dieser Variante wird bei Verwendung von Epichlorhydrin und bei Einsatz von Dihydroxyanthrachinonen bei kurzzeitigem Kochen, z. B. bis 48 Stunden oder auch darüber, in der Regel weitgehend selektiv nur eine der beiden Hydroxylgruppen in den Glycidyläther überführt, während zur Umsetzung der zweiten Hydroxygruppe die Reaktionszeiten wesentlich verlängert werden müssen, bis zu 500 stunden oder darüber. Allerdings verläuft die bifunktionelle Überführung der Dihydroxyanthrachinone in deren Bisglycidyläther mit dem reaktiveren Epibromhydrin überraschenderweise ausbeutemäßig (ca. 60 %) wesentlich besser und auch in kürzerer Reaktionszeit als bei Epichlorhydrin. Darüber hinaus verläuft die Umsetzung mit Epibromhydrin auch vollständiger zu Oxy-2,3-oxidopropangruppen als bei Epichlorhydrin, d. h. es wird weniger oder auch gar kein Primärprodukt der Formel III erhalten. Fällt jedoch im Gemisch mit I noch Primärprodukt III als Reaktionsprodukt an, so wird dieses durch weiteres Rückflußkochen in organischen Lösungsmitteln mit Alkalien oder auch durch Behandeln mit Triäthylamin in Tetrahydrofuran vollständig zum Titelprodukt I unter Eliminierung von Chlor- oder Bromwasserstoff überführt. Eine Reinigung der erhaltenen Verfahrensprodukte kann durch Umkristallisation oder auch durch übliche Chromatographie an Aluminiumoxid oder Kieselgel erfolgen. Auch eine Auftrennung der Reaktionsprodukte in einem Reaktionsgemisch, z. B. von Mono- und Diglycidyläther eines Di- oder Polyhydroxyanthrachinons, erfolgt zweckmäßigerweise durch Chromatographie. Zur Verläufskontrolle der Reaktionsführungen verwendet man die Dünnschichtchromatographie, die eindeutig den Verlauf und den Selektivitätsgrad der Reaktionen, insbesondere bei Verwendung von Dihydroxyanthrachinonen, erkennen läßt. Die $R_F$-Werte von Mono- und Diglycidyläther eines eingesetzten Di- oder Polyhydroxyanthrachinons liegen in der Regel weit auseinander. Die DC-Verlaufskontrolle bewährt sich insbesondere zur Darstellung von Hydroxyanthrachinonoxy-2,3-oxidopropanen, da dann die Reaktion vor Weiterreaktion zu den Anthrachinon-di(oxy-2,3-oxidopropanen) rechtzeitig abgebrochen werden muß. Aber auch zur Kontrolle der Bildung von Anthrachinon-di(oxy-2,3-oxidopropanen) hat sich die Dünnschichtverlaufskontrolle sehr bewährt.

Die eben geschilderten praktischen Durchführungsbedingungen gelten in der Regel auch für analoge, durch Basen bzw. Alkalien bewirkte Ätherbildungsreaktionen, wie sie in den folgenden Verfahrensvarianten erläutert werden.

**Verfahrensvariante 2** (Formel II → III → I)

Durch kurzzeitiges, etwa 1- bis 12-stündiges, Erhitzen von Mono- oder Dihydroxyanthrachinonen gemäß Verfahrensvariante 1 mit Epichlor- und Epibromhydrin werden vorzugsweise die Halogenhydrine der Formel III gebildet. Ferner werden diese auch in guten Ausbeuten erhalten, wenn man die Mono- bzw. Dihydroxyanthrachinone mit 1 bis 3 Mol, vorzugsweise 1,1 bis 1,4 Mol, Epibrom- vorzugsweise Epichlorhydrin mit katalytischen Mengen Piperidin 1 bis 16 Stunden, vorzugsweise 6 - 8 Stunden, am Rückfluß kocht. Durch anschließende Behandlung der Verbindungen der Formel III mit Alkalien, vorzugsweise 1 normaler Natronlauge in Tetrahydrofuran, bei 30 - 70°C bis zu 1 - 3 Stunden, erhält man nach Halogenwasserstoffeliminierung die Verbindungen der Formel I.

**Verfahrensvariante 3** (II + IV → V → I)

Die Umsetzung der Anthrachinone II mit Glycidol IV oder seinen Derivaten ($R_4$ in IV = $CH_3$, $COCH_3$, $SO_2CH_3$ usw.) erfolgt unter den gleichen Bedingungen wie für die Reaktion mit Epichlor- bzw. Epibromhydrin unter Verfahrensvariante 1 beschrieben. Auch hier wird zur Verlaufskontrolle der Reaktionsführung die Dünnschichtchromatographie herangezogen. Zur weiteren Überführung von V in I wird V ($R_4$ = H) vorzugsweise mit Methan- oder p-Toluolsulfonsäurechlorid in Dioxan/Pyridin bei 0°C bis 20°C zu den entsprechenden Sulfonsäureesterderivaten (V : $R_4$ = $SO_2CH_3$, $SO_2C_6H_4$-p-$CH_3$) umgesetzt und diese danach direkt oder nach nucleophilem Umsatz mit Alkalibromid bzw. -jodid in vorzugsweise Dimethylformamid, gegebenenfalls unter Zusatz von Alkalicarbonaten, zu den entsprechenden Halogeniden (V : $R_4$ = Br oder J) umsetzt, die dann durch Kochen mit Alkalien in vorzugsweise Alkoholen oder Aceton oder Butanon-2 oder durch Behandlung mit tertiären organischen Basen, vorzugsweise Triäthylamin, in Äthern, z. B. Tetrahydrofuran oder Dioxan oder Dimethoxyglykol, oder in Xylol unter Epoxidbildung zu den Titelverbindungen der Formel 1 umgesetzt werden.

**Verfahrensvariante 4** (II → VII → I)

Die Hydroxyanthrachinone II werden mit 1-Chlor-, 1-Brom- oder 1-Jod-2,3-propandiol vorzugsweise in Alkoholen oder Dimethylformamid oder Dimethyloxyäthan mit Alkalien, vorzugsweise Alkalialkoholaten bis zur Komplettierung der Reaktion am Rückfluß gekocht. Die weiteren Reaktionen zu I erfolgen analog, wie bei Verfahrensvariante 3 beschrieben.

**Verfahrensvariante 5** (II → VIII → VII → I)

Die Hydroxyanthrachinone II werden mit vorzugsweise 1-Brom, 1-Jod- oder 1-(p-Toluolsulfonato)-2,3-propandiol-2,3-acetonid in analoger Weise wie bei Verfahrensvariante 4 für die freien 1-Halogen-2,3-propandiole beschrieben, zu Verbindungen der Formel VIII umgesetzt und anschließend durch Behandeln mit Mineralsäuren, vorzugsweise Salz- oder Schwefelsäure in Alkoholen bzw. Aceton/Wasser zu Verbindungen der Formel VII hydrolysiert. Die Umsetzung zu den Titelverbindungen I erfolgt analog Verfahrensvariante 4 bzw. 3.

**Verfahrensvariante 6** (II → X → I)

Die Umsetzung von Hydroxyanthrachinonon zu den Mono- bzw. Diallyloxyanthrachinonen X erfolgt in analogen Weise wie unter Verfahrensweise 1 für die Umsetzung von I → II beschrieben. Anstatt Epichlor- oder Epibromhydrin werden vorzugsweise Allylchlorid, Allylbromid oder Allyljodid verwendet.

Zur Überführung von X → I werden die Anthrachinonallyläther X in inerten organischen Lösungsmitteln, vorzugsweise Dichlormethan, Chloroform, Essigester mit entsprechenden Moläquivalenten einer organischen Alkyl- oder Arylpercarbonsäure, vorzugsweise mit m-Chlorperbenzoesäure bei 0°C -40°, umgesetzt, wobei in der Regel m-Chlorbenzoesäure ausfällt, die abfiltriert wird. Aus dem Filtrat können dann die erfindungsgemäßen Verbindungen der Formel I durch Einengen und Umkristallisieren gewonnen werden.

In einer alternativen Variante können die Verfahrensprodukte I aus den Anthrachinonallyläthern auch durch Umsetzung mit dem Oxydationskomplex aus Jod und Silberoxid ($J_2 \cdot Ag_2O$) in Wasser/Dioxan erhalten werden.

Das selektive, zu den erfindungsgemäßen Produkten führende Reaktionsverhalten muß als überraschend angesehen werden.

Folgende Hydroxyanthrachinone, die in der Regel literaturbekannt sind, kommen als Ausgangsverbindungen für die Herstelung der erfindungsgemäßen Anthrachinon-mono- oder bis-(oxy-2,3-oxido-propane) I in Betracht

1- oder 2-Hydroxyanthrachinon;
1,5-, 1,6-, 1,7-, 1,8-, 2,6
und 2,7-, 1,2-, 1,3-, 1,4- und 2,4-Dihydroxyanthrachinon, ferner
Tri- und Tetrahydroxyanthrachinone
wobei diese Anthrachinone zusätzlich einen oder mehrere gleiche oder verschiedene Substituenten, die vorzugsweise Halogen, Alkyl, Alkoxy, Acyloxy oder Nitro bedeuten, enthalten können.

Als erfindungsgemäße Verfahrensprodukte werden aus den Ausgangsanthrachinonen folgende Glycidyläther erhalten:

Anthrachinon-1-oxy-(2,3-oxido-propan)
Anthrachinon-2-oxy-(2,3-oxido-propan)
1-Hydroxyanthrachinon-5-oxy-(2,3-oxido-propan)

| | | | | | | |
|---|---|---|---|---|---|---|
| 5- | " | " | -1- | " | " |
| 1- | " | " | -6- | " | " |
| 6- | " | " | -1- | " | " |
| 1- | " | " | -7- | " | " |
| 7- | " | " | -1- | " | " |
| 1- | " | " | -8- | " | " |
| 8- | " | " | -1- | " | " |
| 2- | " | " | -6- | " | " |
| 6- | " | " | -2- | " | " |
| 2- | " | " | -7- | " | " |
| 7- | " | " | -2- | " | " |

Anthrachinon-1,5-bis-(oxy-2,3-oxido-propan)

| | | | | | |
|---|---|---|---|---|---|
| " | -1,6 | " | " | " | " |
| " | -1,7 | " | " | " | " |
| " | -1,8 | " | " | " | " |
| " | -2,6 | " | " | " | " |
| " | -2,7 | " | " | " | " |
| " | -1,2 | " | " | " | " |
| " | -1,3 | " | " | " | " |
| " | -1,4 | " | " | " | " |
| " | -2,3 | " | " | " | " |

wobei diese Verfahrensprodukte zusätzlich einen oder mehrere gleiche oder verschiedene Substituenten, die vorzugsweise Halogen, Alkyl, Alkoxy, Hydroxy, Acyloxy oder Nitro bedeuten, enthalten können.

Die gemäß der Erfindung erhältlichen Verfahrensprodukte stellen wertvolle Zwischenprodukte für die Herstellung von Herz-Kreislaufmitteln vom Strukturtyp der β-Rezeptorenblocker dar. Ferner können die Anthrachinon-bis-(oxy-2,3-oxido-propane) als neuartige bisher nicht bekannt gewordene quervernetzende Agenzien bei der Herstellung von Polymeren, z. B. Epoxydharzen und -lacken, in der Kunststoff- und Lackbranche eingesetzt werden.

Darüberhinaus zeigen die Verfahrensprodukte überraschenderweise in verschiedenen in vitro und auch in vivo Testmodellen eine dem Adriamycin vergleichbare oder oft auch überlegene antitumorale Wirksamkeit. Die akute Toxizität der erfindungsgemäßen Verbindungen ist dabei signifikant niedriger als die des Vergleichstandard Adriamycin, d. h. die Verfahrensprodukte zeigen einen größeren therapeutischen Index als Adriamycin. Die unterschiedlichen Testsysteme zur Bestimmung der in vitro bzw. der in vivo Antitumorwirkung sowie der akuten Toxizität werden im folgenden Abschnitt beschrieben.

a) Ermittlung der zytotoxischen Aktivität in vitro

Die Bestimmung der zytotoxischen Wirksamkeit der in diesem Patent beschriebenen Verbindungen erfolgt an L 1210 Leukämiezellen der Maus. Im einzelnen wurden folgende Testsysteme verwendet:

**Proliferationsassay**

Bei dieser Methode wird in vitro nach Inkubation der Zellen mit unterschiedlichen Konzentrationen der Testsubstanz ermittelt, inwieweit die Zellen radioaktiv markierte DNA-Vorläufer (z. B. C 14 markiertes Thymidin) einbauen können.

L 1210 Zellen in expotentieller Wachstumsphase ($5 \times 10^3$/ml in RPMI1640) werden in einer Mikrotiterplatte 72 Stunden mit unterschiedlichen Konzentrationen der Testsubstanz inkubiert (37°C, 5 % $CO_2$, 95 % relative Luftfeuchte). Kontrollen bestehen aus Zellen, die lediglich mit frischem Medium inkubiert werden. Alle

Bestimmungen werden als 4-fach Bestimmungen durchgeführt. Nach 65 Stunden werden 50 µl C 14 Thymidin (1,5 µc/ml) zugegeben, um die DNA der Zelle radioaktiv zu markieren. Nach 7 Stunden Inkubation werden die Zellen abgesaugt, die DNA mit 5-%-iger Trichloressigsäure gefällt und nacheinander mit Wasser bzw. Methanol gewaschen.

Nach Trocknung bei 50°C wird die in die DNA eingebaute Radioaktivität nach Zugabe von 5 ml Szinstilationsflüssigkeit ermittelt.

Die Ergebnisse werden angegeben als Verhältnis der Szinstilationsindices nach Inkubation mit der Testsubstanz bezogen auf die unbehandelte Kontrolle. Aus den so erhaltenen Meßwerten wird die Dosiswirkungskurve ermittelt und grafisch die $IC_{50}$, d. h. die Konzentration, die unter Testbedingungen den Einbau von radioaktivem Thymidin um 50 % gegenüber der Kontrolle erniedrigt ermittelt. Die $IC_{50}$ Werte der in diesem Patent beschriebenen Verbindungen im Vergleich zu Adriamycin (ADM) werden in der Tabelle 1 zusammengefaßt.

b) Koloniebildung von L 1210 Leukämiezellen in soft agar

Diese Methode dient zum Nachweis eines Einflusses der Testsubstanzen auf das Wachstumsverhalten der Zellen über mehrere Generationen (bei einer Zellzykluszeit von 10 - 12 Stunden werden in der Testzeit von 7 Tagen ca. 14 aufeinanderfolgende Generationen beobachtet). Zytostatisch wirksame Substanzen bewirken in diesem Test eine Reduktion der zu beobachtenden Koloniezahl gegenüber einer unbehandelten Kontrolle. Im einzelnen wird der Test wie folgt durchgeführt:

500 Leukämiezellen pro Platte werden mit unterschiedlichen Konzentrationen von Testsubstanz 1 Stunde bei 37°C inkubiert. Anschließend werden die Zellen zweimal mit McCoy5a Medium gewaschen und schließlich in Petrischalen nach Zusatz von 0,3 % Agar ausgegossen. Kontrollen werden lediglich mit frischem Medium inkubiert. Anstelle der 1-stündigen Inkubation werden in manchen Fällen unterscheidliche Konzentrationen und Testsubstanz der oberen Agarschicht zugemischt, um so eine kontinuierliche Exposition der Zellen über die gesamte Inkubationszeit zu erreichen. Nach Erstarren des Agars werden die Platten im Brutschrank 7 Tage bei 37°C inkubiert (5 % $CO_2$, 95 % relative Luftfeuchtigkeit).

Anschließend wird die Anzahl der entstandenen Kolonien mit einem Durchmesser > 60 µm gezählt. Die Ergebnisse werden angegeben als Koloniezahl in behandelten Agarplatten in Prozent der unbehandelten Kontrolle. Aus der so erhaltenen Dosiswirkungskurve wird die $IC_{50}$ als Maß für die Wirksamkeit der Substanz ermittelt. Die Ergebnisse für die hier beschriebenen Verbindungen im Vergleich zu Adriamycin sind in Tabelle 1 zusammengefaßt.

Die Tabelle 1 zeigt, daß die Verfahrensprodukte überraschenderweise unter in vitro Bedingungen an L 1210 Leukämiezellen der Maus eine dem Adriamycin vergleichbare oder in Abhängigkeit von der Struktur oft auch eine überlegene Antitumorwirksamkeit besitzen.

**Ermittlung der akuten Toxizität**

Zur Ermittlung der akuten Toxizität wurden NMRI Mäusen am Tag 0 unterschiedliche Dosen der Testsubstanz suspendiert in 0,5 ml, 0,5-%-iger Carboxymethylcellulose intraperitoneal injiziert. Kontrollgruppen erhielten lediglich 0,5 ml der Carboxymethylcellulose-Lösung. Pro Konzentration an Testsubstanz wurden 5 Mäuse eingesetzt. Am Tage 14 wird die Zahl der überlebenden Mäuse ermittelt und daraus nach der Litchfield Wilcoxon Methode die LD 50 ermittelt. Die Toxizität der in diesem Patent beschricbenen Verbindungen im Vergleich zu Adriamycin wird in Tabelle 1 zusammengestellt.

**Tabelle 1:-**

| Substanz | Proliferationsassay $IC_{50}$ (µug/ml) | Stammzellassay $IC_{50}$ (µg/ml) Inkubition: Kontin./1 h | | Akute Toxizität (mg/kg) $LD_{50}$ |
|---|---|---|---|---|
| Adriamycin | $6 \times 10^{3}$ | $2 \times 10^{-2}$ | $4,4 \times 10^{-2}$ | 14 |
| Beisp. 5 | $4,4 \times 10^{-3}$ | $1 \times 10^{-2}$ | $8,3 \times 10^{-3}$ | 200 |
| Beisp. 4 | $2,2 \times 10^{-3}$ | $3,3 \times 10^{-3}$ | $4 \times 10^{-3}$ | |
| Beisp. 3 | $2,8 \times 10^{-2}$ | $3 \times 10^{-2}$ | $6,6 \times 10^{-2}$ | |
| Beisp. 8 | $2,8 \times 10^{-2}$ | $8,5 \times 10^{-3}$ | $3 \times 10^{-2}$ | |

**Beschreibung der Versuche**

Schmelzpunkte: Apparat nach Tottoli (Fa. Büchi), nicht korrigiert.
IR-Spektrum (in KBr): Gitterspektrophotometer Perkin-Elmer 521.
Es werden jeweils nur charakteristischen Banden angeführt.
- UV-Spektren (in Methanol): spektralphotometer Beckman DK 1A.

- $^1H$-NMR-Spektren (wenn nicht anders erwähnt), in $CDCl_3$ Tetramethylsilan als innerer Standard): Varian A 60 oder T 60.

Massenspektren (MS): Gerät MS9 (Fa. AEI).

- Dünnschichtchromatographie (DC): Fertigplatten Kieselgel F254 (Fa. Merck).

Als Fließmittel wurde, wenn nicht anders angeführt, $CH_2Cl_2$/Essigester = 4 : 1, verwendet (Laufstrecke 15 cm; 1-mal entwickelt). Die Sichtbarmachung der Flecke erfolgt, wenn eine Identifizierung durch die Eigenfärbung der Anthrachinonverbindungen nicht exakt gewährleistet war, durch Bestrahlung mit UV-Licht.

Für die Säulenchromatographie wird, wenn nicht anders angegeben, Aluminiumoxid der Fa. Woelm, neutral, Aktivitätsstufe II, verwendet. - Wegen der Empfindlichkeit der Epoxidgruppen wurde stets <u>streng säurefreies</u> Methylenchlorid verwendet.

**Beispiel 1**

Anthrachinon-2-(oxy)-2,3-oxido-propan)

<u>a</u>. In einer am Wasserabscheider lebhaft unter Rückfluß siedenden Lösung oder Suspension von 10 g 2-Hydroxy-anthrachinon in 100 ml Epichlorhydrin (Badtemperatur ca. 150°) wird innner-halb von 30 Minuten eine Lösung von 3 g Natriumhydroxid in 7 ml Wasser unter starkem Rühren zugetropft. Anschließend wird 8 Stunden weiterhin unter Rückfluß und starkem Rühren am Wasserabscheider zum lebhaften Sieden erhitzt. Es wird von ausgefallenem Natriumchlorid abgesaugt. Der Filterrückstand wird mit ca. 100 ml säurefreiem Methylenchlorid gewaschen. Die vereinigten Filtrate werden zunächst unter leicht vermindertem Druck (80 - 100 mm Hg) von Lösungsmittel und überschüssigen Epichlorhydrin befreit. Anschließend wird der Destillationsrückstand in Methylenchlorid gelöst und über eine Säule von Aluminiumoxid (h = 10 cm, Ø = 3 cm) filtriert, wobei etwa 500 ml Methylenchlorid als Eluationsmittel verwendet werden. Nach dem Abdestillieren der Lösungsmittel wird der Rückstand aus Methylenchlorid/Methanol und Zusatz von Diäthyläther zum Zwecke der vollständigen Auskristallisation umkristalliert. Man erhält 4,2 g Anthrachinon-2-(oxy-2,3-oxido-propan) vom Schmp. 194 - 197°C.

MS: m/e = 280 (M+)

<u>IH-NMR-Spektrum</u> (in DMSO):

Charakteristisches Signalmuster für $-OCH_2\overset{O}{CH - CH_2}$ Gruppe:

δ = 2,8 ppm (AB-Teil eines ABX-Spektrums, <u>$CH_2$</u>-1)
δ = 3,4 ppm (m, C<u>H</u>-2)
δ = 4,3 ppm (AB-Teil eines ABX-Spektrums, <u>$CH_2$</u>-3)

<u>IR-Spektrum:</u> Banden bei 3080, 1675, 1590, 1580, 1500, 1330, 1300, 1240, 1150, 1030, 935, 850, 775, 715 $cm^{-1}$
<u>DC</u> (Laufsmittel $CH_2Cl_2$ : $CH_3OH$ = 19 : 1)
$R_F$ = 0,6
(Ausgangsmaterial: $R_F$ = 8,0)

<u>b</u>. 3,5 g 2-Hydroxyanthrachinon werden in 29 ml Dioxan mit 21,9 ml Epidbromhydrin und 29 ml 1-N wässriger Natronlauge versetzt und 2,5 Stunden bei 50°C gerührt. Danach wird eingeengt und der Rückstand mehrmals mit Methylenchlorid ausgezogen. Nach analoger Weiterbehandlung und Isolierung der Substanz, wie unter Beispiel 1a. angegeben, werden 1,2 g Anthrachinon-2-(oxy-2,3-oxido-propan) mit den gleichen physikalischen und spektralen Daten wie dort angegeben erhalten.

**Beispiel 2**

Anthrachinon-1-(oxy-2,3-oxido-propan)

Eine Lösung von 27 g 1-Hydroxy-anthrachinon in 630 ml Äthylmethylketon wird mit 27 g Kaliumcarbonat sowie 135 ml Epibromhydrin versetzt und 100 Stunden unter Rühren am Rückfluß erhitzt. Danach filtriert man über ein Klärschichtfilter ab, engt i. Vak. das Filtrat ein und kristallisiert aus Äthylmethylketon unter Zusatz von Methylenchlorid zur vollständigen Auflösung um und man erhält 13,5 g Anthrachinon-1-(oxy-2,3-oxido-propan) vom Schmp. 177 - 180°C.

<u>MS:</u> m/e = 280 (M+)

<u>$^1$H-NMR-Spektrum</u>

Charakteristisches Siganlmuster für $-O-CH_2\overbrace{CH - CH_2}^{O}$ Gruppe:

$\delta$ = 3,0 ppm (AB-Teil eines ABX-Spektrums, $C\underline{H}_2$-3)
$\delta$ = 3,45 ppm (m, $C\underline{H}$-2)
$\delta$ = 4,3 ppm (AB-Teil eines ABX-Spektrums, $C\underline{H}_2$-1)
Das Aromatenbandemuster liegt zwischen 7,25 und 8,3 ppm.

**Beispiel 3:**

1-Hydroxy-anthrachinon-4-(oxy-2,3-oxido-propan)

a. Eine Lösung von 20 g 1,4-Dihydroxyanthrachinon in 500 ml Äthylmethylketon wird mit 20,5 g Kaliumcarbonat und 100 ml Epichlorhydrin versetzt und 20 Stunden unter Rühren am Rückfluß erhitzt. Danach filtriert man von dem unlöslichen Anteilen ab und sengt das Filtrat i. Vak. ein. Der Rückstand wird mehrmals aus Aceton (eventuell etwas Methylenchloridzugabe zur vollständigen Auflösung) umkristallisiert und ergibt 11,2 g ziegelrot gefärbtes 1-Hydroxy-anthrachinon-4-(oxy-2,3-oxido-propan) vom Schmp. 148° C.
MS: m/e = 296 (M+) (kein Molpeak für das Bisepoxid gemäß Beispiel 4 - bei m/e = 352 (M+)).
$^1$H-NMR-Spektrum ($DCCl_3$):

Charakteristisches Signalmuster für $-O-CH_2\overbrace{CH - CH_2}^{O}$ Gruppe:

$\delta$ = 2,9 ppm (AB-Teil eines ABX-Spektrums, $C\underline{H}_2$-3)
$\delta$ =3, 4 ppm (m, $C\underline{H}$-2)
$\delta$ = 4,3 ppm (AB-Teil eines ABX-Spektrums, $C\underline{H}_2$-1)

DC: $R_F$ = 0,8 ($R_F$-Ausgangsmaterial: 0,95)

b. Wird in gleicher Weise, wie unter Beispiel 3a. angegeben, eine Lösung von 20 g 1,4-Dihydroxyanthrachinon in 500 ml Äthylmethylketon oder Aceton mit 20,5 g Kaliumcarbonat und 50 ml Epibromhydrin 8 Stunden unter Rühren am Rückfluß gekocht, weiter behandelt und aufgearbeitet, so erhält man nach nochmaligen Umkristallisieren aus Aceton das gleiche Reaktionsprodukt mit den gleichen Daten, wie unter Beispiel 3a. angegeben.

Allerdings kann im zuerst gewonnenen Rohprodukt aufgrund der DC bereits das in Beispiel 4 beschriebene 1,4-Bisepoxid als Nebenprodukt identifiziert werden, das aber durch fraktioniertes Umkristallisieren aus Aceton oder auch durch Chromatographie an neutralen Aluminiumoxid vom hier gewünschten Monoepoxid gemäß Beispiel 3a.) abgetrennt wird.

DC:
1. gereinigtes Verfahrensprodukt: $R_F$ = 0,8. (keine weiteren Flecke sichtbar)
2. Rohprodukt:
   Hauptfleck $R_F$ = 0,8
   Nebenfleck $R_F$ = 0,4
3. Ausgangsmaterial $R_F$ = 0,95

**Beispiel 4:**

Anthrachinon-1,4-bis-(oxy-2,3-oxido-propan)

Eine Lösung von 60 g 1,4-Dihydroxyanthrachinon in 150 ml Äthylmethylketon wird mit 61,5 g Kaliumcarbonat sowie 300 ml Epibromhydrin versetzt und 100 Stunden unter gutem Rühren am Rückfluß gekocht. Man läßt auf Zimmertemperatur abkühlen und filtriert die unlöslichen Anteile ab. Das Filtrat wird i. Vak. eingeengt und das resultierende Öl durch Anreiben mit ca. 1 l Diäthyläther zur Kristallisation gebracht. Man filtert ab, wäscht das Kristallisat mehrfach Diäthyläther nach, trocknet es (64 g schmutziggelbgrünes Kristallisat, das aufgrund des DC nur noch mäßige Verunreinigungen zeigt) und löst dieses in ca. 200 - 300 ml säurefreiem Methylenchlorid. Diese Lösung wird auf eine Säule, die mit Aluminiumoxid, Woelm, neutral, AKt.-St. II (h = 10 cm, Ø = 8,5 cm) gefüllt ist, aufgezogen. Nach Durchsatz von ca. 200 ml Vorlauf, der nicht verwendet wird, wird mit säurefreiem Methylenchlroid eluiert, bis eine Probe keine nenneswerten Anteile an Reaktionsprodukt mehr zeigt (Durchsatz etwa 1,5 - 4 l Eluationsmittel). Man destilliert das Eluationsmittel i. Vak. ab und kristallisiert den kristallinen,

gelben Rückstand aus Aceton/Methylenchlorid (Lösen in der Siedehitze und Abdestillieren des Methylenchlorids) und Zusatz von Diäthyläther zur noch warmen Lösung um. Nach dem Abfiltrieren und Waschen mit Diäthyläther erhält man nach dem Trocknen etwa 20 g Anthrachinon-1,4-bis-(oxy-2,3-oxido-propan) vom Schmp. 168°C

$^1$H-NMR-Spektrum ($DCCl_3$):

O

Characteristisches Signalmuster für $-OCH_2CH - CH_2$ Gruppe:

$\delta = 2{,}8$ ppm (AB-Teil eines ABX-Spektrums, $CH_2$-3)
$\delta = 3{,}34$ ppm (m, CH-2)
$\delta = 4{,}3$ ppm (AB-Teil eines ABX-Spektrums, $CH_2$-1)

MS: m/e = 352 (M+)
DC: $R_F = 0{,}4$

**Beispiel 5:**

Anthrachinon-2,6-bis(oxy-2,3-oxido-propan)

a. Eine Lösung bzw. Suspension von 40 g 2,6-Dihydroxy-anthrachinon in 1 000 ml Butanon-(2) wird mit 40,8 g Kaliumcarbonat und 200 ml Epibromhydrin versetzt und 110 Stunden unter Rühren am Rückfluß erhitzt. Man filtriert von dem unlöslichen Anteilen ab, engt i. Vak. das Filtrat ein, löst den Rückstand in Äthylmethylketon, ggf. unter Zusatz von Methylenchlorid, behandelt kurz mit Aktiv-Kohle, filtriert und läßt das Reaktionsprodukt auskristallisieren, nachdem man ein Teil der Lösungsmittel abdestilliert hat. Man kristallisiert zweckmäßigerweise noch mehrmals aus Butanon-(2) und/oder Aceton um, und erhält 5 bis 10 g hellgelbgefärbtes Anthrachinon-2,6-bis-(oxy-2,3-oxido-propan) vom Schmp. 188 - 191°C. Dieses Reaktionsprodukt fällt während der Umkristallisation zuweilen auch in einer höherschmelzenden Form, nämlich mit dem Schmp. 218 - 220°C an.

b. Die oben angefallene abfiltrierten umlöslichen Anteile werden mit absolutem Aceton am Soxleth-Extraktionsgefäß erschöpfend extrahiert. Das nach dem Erkalten des Extraktionsmittels ausgefallene gelbgrüne Kristallisat wird abfiltriert (ca. 20 g) und erneut, jetzt mit absolutem Methylenchlorid, am Soxleth-Extraktionsgerät erschöpfend extrahiert. Nach dem Abdestillieren des Lösungsmittels wird aus Aceton oder Butanon-(2), unter Zusatz von Methylenchlorid, umkristallisiert. Man erhält etwa 10 g der Titelverbindung vom Schmp. 218 - 220°C.

Beide Formen der Titelverbindung, sowohl die niedrig- als auch die hochschmelzenden Form, zeigen identische IR-, NMR- und MS-Spektren sowie einen identischen $R_F$-Wert in Dünnschichtchromatogramm.

MS: m/e = 352 (M+)

$^1$H-NMR-Spektrum (in DMSO):

O

Charakteristisches Signalmuster für $-OCH_2CH - CH_2$-Gruppe:

$\delta = 2{,}8$ ppm (AB-Teil eines ABX-Spektrums, $CH_2$-1)
$\delta = 3{,}4$ ppm (m, CH-2)
$\delta = 4{,}3$ ppm (AB-Teil eines ABX-Spektrums, $CH_2$-3)
DC $R_F = 0{,}78$ ($R_F$ Ausgangsmaterial = 0,37)

c. In einem zweiten analog durchgeführten Reaktionsansatz läßt man das Reaktionsgemisch nach 110 Stunden Reaktionszeit auf Zimmertemperatur abkühlen, filtriert von Ungelösten ab und extrahiert dieses wie unter b. angegebenen, an der Soxleth-Extrationsapparatur mit Aceton, und nach Isolierung des bei 20°C ausgefallenen Kristallisats wird letzteres, wie unter b. angegeben, mit Methylenchlorid am Soxleth extrahiert. Nach analoger Isolierungstechnik, wie bei b. angegebenen, werden 24 g Anthrachinon-2,6-bis-(oxy-2,3-oxido-propan) vom Schmp. 219 - 222°C erhalten, das in allen spektralen Daten und im $R_F$-Wert im DC mit dem nach a. und b. erhaltenen Reaktionsprodukt vollkommen identisch ist.

**Beispiel 6:**

Anthrachinon-2,7-bis-(oxy-2,3-oxido-propan)

Eine Lösung von 30 g 2,7-Hydroxy-anthrachinon in 700 ml Äthylmethylketon wird mit 30,6 g Kaliumcarbonat sowie 150 ml Epibromhydrin vrsetzt und 120 Stunden unter Rühren am Rückfluß erhitzt. Danach filtriert man (noch heiß) über ein Klärschichtfilter ab, engt in Vak. das Filtrat ein, chromatographiert den Rückstand mit Methylenchlorid gemäß Beispiel 4 an Aluminiumoxid, und kristallisiert aus Äthylmethylketon unter Zusatz von Methylenchlorid zur vollständigen Auflösung, um und man erhält 10,8 g Anthrachinon-2,7-bis-(oxy-2,3-oxido-propan) vom Schmp. 208 - 212°C

MS: m/e = 352 (M+)

$^1$H-NMR-Spektrum:

O

Charakteristisches Signalmuster für $-O-CH_2CH-CH_2$ Gruppe:

δ = 2,8 ppm (AB-Teil eines ABX-Spektrums, $CH_2$-3)
δ = 3,45 ppm (m, CH-2)
δ = 4,3 ppm (AB-Teil eines ABX-Spektrums, $CH_2$-1)

**Beispiel 7:**

Anthrachinon-1,2-bis-(oxy-2,3-oxido-propan)

Ein Lösung von 30 g 1,2-Dihydroxy-anthrachinon in 700 ml Äthylmethylketon wird mit 30,6 g Kaliumcarbonat sowie 150 ml Epibromhydrin versetzt und 100 Stunden unter Rühren am Rückfluß erhitzt. Danach filtriert man (noch heiß) ab, engt i. Vak. das Filtrat ein und kristallisiert aus Äthylmethylketon, ggf. unter Zusatz von Methylenchlorid zur vollständigen Auflösung um und man erhält 13,0 g Anthrachinon-1,2-bis-(oxy-2,3-oxido-propan) vom Schmp. 220°C.

MS: m/e = 352 (M+)

$^1$H-NMR-Spektrum:

O

Charakteristisches Signalmuster für $-O-CH_2CH-CH_2$ Gruppe:

δ = 2,8 ppm (AB-Teil eines ABX-Spektrums, $CH_2$-3)
δ = 3,45 ppm (m, CH-2)
δ = 4,3 ppm (AB-Teil eines ABX-Spektrums, $CH_2$-1).

**Beispiel 8:**

Anthrachinon-1,8-bis-(oxy-2,3-oxido-propan)

Eine Lösung von 30 g 1,8-Dihydroxy-anthrachinon in 700 ml Äthylmethylketon wird mit 30,6 g Kaliumcarbonat sowie 150 ml Epibromhydrin versetzt und 100 Stunden unter Rühren am Rückfluß erhitzt. Danach filtriert man (noch heiß) ab, engt i. Vak. das Filtrat ein und kristallisiert aus Äthylmethylketon, ggf. unter Zusatz von Methylenchlorid zur vollständigen Auflösung um und man erhält 10,5 g Anthrachinon-1,8-bis-(oxy-2,3-oxid-propan) vom Schmp. 187 - 189°C.

MS: m/e = 352 (M+)

$^1$H-NMR-Spekt rum:

O

Charakteristisches Signalmuster für $-O-CH_2CH-CH_2$ Gruppe:

δ = 2,8 ppm (AB-Teil eines ABX-Spektrums, $CH_2$-3)
δ = 3,45 ppm (m, CH-2)
δ = 4,3 ppm (AR-Teil eines ABX-Spektrums, $CH_2$-1).

**Beispiel 9:**

Anthrachinon-1,5-bis-(oxy-2,3-oxido-propan)

Eine Lösung von 30 g 1,5-Dihydroxy-anthrachinon in 700 ml Äthylmethylketon wird mit 30,6 g Kaliumcarbonat sowie 150 ml Epibromhydrin versetzt und 100 Stunden unter Rühren am Rückfluß erhitzt. Danach filtriert man (noch heiß) ab, engt i. Vak. das Filtrat ein, chromatographiert gegebenenfalls den Rückstand mit Methylenchlorid als Aufzieh- und Eluationsmittel gemäß Beispiel 4 an Aluminiumoxid und kristallisiert aus Äthylmethylketon, ggf. unter Zusatz von Methylenchlorid zur vollständigen Auflösung um und man erhält 11,0 g Anthrachinon-1,5-bis-(oxy-2,3-oxido-propan) vom Schmp. 205° C.

MS: m/e = 352 (M+)

$^1H$-NMR-Spektrum:

Charakteristisches Signalmuster für $-O-CH_2\overbrace{CH-CH_2}^{O}$ Gruppe:

δ = 2,8 ppm (AB-Teil eines ABX-Spektrums, $CH_2$-3)
δ = 3,45 ppm (m, CH-2)
δ = 4,3 ppm (AB-Teil eines ABX-Spektrums, $CH_2$-1).

**Beispiel 10**

Anthrachinon-6-methoxycarbonyl-1,4-bis-(oxy-2,3-oxido-propan)

Eine Lösung von 3 g 1,4-Dihydroxy-6-methoxycarbonyl-anthrachinon in 70 ml (absol.) Äthylmethylketon wird mit 3 g Kaliumcarbonat sowie 15 ml Epibromhydrin versetzt und 100 Stunden unter Rühren am Rückfluß unter strengem Feuchtigkeitsausschluß erhitzt. Danach filtriert man (noch heiß) ab, engt i. Vak. das Filtrat ein und kristallisiert aus Äthylmethylketon, ggf. unter Zusatz von Methylenchlorid zur vollständigen Auflösung um und man erhält 1,2 g amorphes Anthrachinon-6-methoxycarbonyl-1,4-bis-(oxy-2,3-oxido-propan).

MS: m/e = 410 (M+)

$^1H$-NMR-Spektrum:

Charakteristisches Signalmuster für $-O-CH_2\overbrace{CH-CH_2}^{O}$ Gruppe:

δ = 2,8 ppm (AB-Teil eines ABX-Spektrums, $CH_2$-3)
δ = 3,45 ppm (m, CH-2)
δ = 4,3 ppm (AB-Teil eines ABX-Spektrums, $CH_2$-1).

**Beispiel 11:**

Anthrachinon-3-methyl-1,8-bis-(oxy-2,3-oxido-propan)

Eine Lösung von 3 g 1,8-Dihydroxy-3-methyl-anthrachinon in 70 ml Äthylmethylketon wird mit 3 g Kaliumcarbonat sowie 15 ml Epibromhydrin versetzt und 100 Stunden unter Rühren am Rückfluß erhitzt. Danach filtriert man (noch heiß) ab, engt i. Vak. das Filtrat ein und kristallisiert aus Äthylmethylketon, ggf. unter Zusatz von Methylenchlorid zur vollständigen Auflösung um und man erhält 1,6 g Anthrachinon-3-methyl-1,8-bis-(oxy-2,3-oxido-propan) vom Schmp. 198 - 209° C.

MS: m/e = 368 (M+)

$^1H$-NMR-Spektrum:

Charakteristisches Signalmuster für $-O-CH_2\overbrace{CH-CH_2}^{O}$ Gruppe:

δ = 2,8 ppm (AB-Teil eines ABX-Spektrums, $CH_2$-3)
δ = 3,45 ppm (m, CH-2)
δ = 4,3 ppm (AB-Teil eines ABX-Spektrums, $CH_2$-1)

**Beispiel 12:**

Anthrachinon-5,8-dichlor-1,4-bis-(oxy-2,3-oxido-propan)

Eine Lösung von 3 g 1,4-Dihydroxy-5,8-dichlor-anthrachinon in 70 ml Äthylmethylketon wird mit 3 g Kaliumcarbonat sowie 15 ml Epibromhydrin versetzt und 100 Stunden unter Rühren am Rückfluß erhitzt. Danach filtriert man (noch heiß) ab, engt i. Vak. das Filtrat ein und kristallisiert aus Äthylmethylketon, ggf. unter Zusatz von Methylenchlorid zur vollständigen Auflösung um und man erhält 1,1 g Anthrachinon-5,8-dichlor-1,4-bis-(oxy-2,3-oxido-propan) vom Schmp. 201 - 212°C.

MS: m/e = 421 (M+)

$^1$H-NMR-Spektrum:

Charakteristisches Signalmuster für 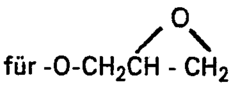 Gruppe:

δ = 2,8 ppm (AB-Teil eines ABX-Spektrums, $CH_2$-3)
δ = 3,45 ppm (m, CH-2)
δ = 4,3 ppm (AB-Teil eines ABX-Spektrums, $CH_2$-1)

**Beispiel 13:**

Anthrachinon-2-acetoxy-1,4-bis-(oxy-2,3-oxido-propan)

Eine Lösung von 3 g 1,4-Dihydroxy-2-acetoxy-anthrachinon in 70 ml Äthylmethylketon wird mit 3 g Kaliumcarbonat sowie 15 ml Epibromhydrin versetzt und 100 Stunden unter Rühren am Rückfluß erhitzt. Danach filtriert man ab, engt i. Vak. das Filtrat ein und kristallisiert aus Äthylmethylketon, ggf. unter Zusatz von Methylenchlorid zur vollständigen Auflösung um und man erhält 1,0 g amorphes Anthrachinon-2-acetoxy-1,4-bis-(oxy-2,3-oxido-propan).

MS: m/e = 410 (M+)

$^1$H-NMR-Spektrum:

Charakteristischea Signalmuster für 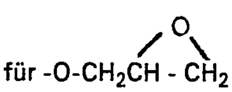 Gruppe:

δ = 2,8 ppm (AB-Teil eines ABX-Spektrums, $CH_2$-3)
δ = 3,45 ppm (m, CH-2)
δ = 4,3 ppm (AB-Teil eines ABX-Spektrums, $CH_2$-1)

**Beispiel 14:**

Anthrachinon-5-nitro-1,4-bis-(oxy-2,3-oxido-propan)

Eine Lösung von 3 g 1,4-Dihydroxy-5-nitro-anthrachinon in 70 ml Äthylmethylketon wird mit 3 g Kaliumcarbonat sowie 15 ml Epibromhydrin versetzt und 100 Stunden unter Rühren am Rückfluß erhitzt. Danach filtriert man (noch heiß) ab, engt i.Vak. das Filtrat ein und kristallisiert aus Äthylmethylketon, ggf. unter Zusatz von Methylenchlorid zur vollständigen Auflösung um und man erhält 1 g Anthrachinon-5-nitro-1,4-bis-(oxy-2,3-oxid-propan) vom Schmp. 171 - 182°C.

MS: m/e = 401 (M+)

$^1$H-NMR-Spektrum:

Charakteristische Signalmuster für 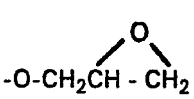 Gruppe:

δ = 2,8 ppm (AB-Teil eines ABX-Spektrums, $CH_2$-3)
δ = 3,45 ppm (m, CH-2)
δ = 4,3 ppm (AB-Teil eines ABX-Spektrums, $CH_2$-1)

**Beispiel 15:**

Anthrachinon-3-methoxv-1,8-bis-(oxy-2,3-oxido-propan)

Eine Lösung von 3 g 1,8-Dihydroxy-3-methoxy-anthrachinon in 70 ml Äthylmethylketon wird mit 3,0 g Kaliumcarbonat sowie 15 ml Epibromhydrin versetzt und 100 Stunden unter Rühren am Rückfluß erhitzt. Danach filtriert man (noch heiß) ab, engt i. Vak. das Filtrat ein und kristallisiert aus Äthylmethylketon, ggf. unter Zusatz von Methylenchlorid zur vollständigen Auflösung um und man erhält 1,0 g Anthrachinon-3-methoxy-1,8-bis-(oxy-2,3-oxido-propan) vom Schmp. 160 - 185° C (unscharf).

MS: m/e = 382 (M+)

$^1$H-NMR-Spektrum:

Charakteristisches Signalmuster für 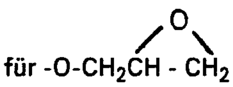 Gruppe:

δ = 2,8 ppm (AB-Teil eines ABX-Spektrums, $CH_2$-3)
δ = 3,45 ppm (m, CH-2)
δ = 4,3 ppm (AB-Teil eines ABX-Spektrums, $CH_2$-1)

**Beispiel 16:**

Anthrachinon-hydroxy-bis-(oxy-2,3-oxido-propan)

Eine Lösung von 6 g 1,4,6-Trihydroxy-anthrachinon in 150 ml Äthylmethylketon wird mit 6,15 g Kaliumcarbonat sowie 30 ml Epibromhydrin versetzt und solange unter gutem Rühren am Rückfluß gekocht, bis eine entnommene Probe im MS einen deutlichen Molekulargewichtspeak bei m/e ≅ 368 (M+) zeigt. Die höhermolekularen MS-Peaks bei > 368 sollten vergleichsweise klein ausfallen. Die Reaktionszeit beträgt hierbei in der Regel 2 bis 4 Tage.

Man läßt auf Zimmertemperatur abkühlen und filtriert die unlöslichen Anteile ab. Das Filtrat wird i. Vak. eingeengt und das resultierende Öl durch Anreiben mit ca. 20 ml Diäthyläther zur Kristallisation gebracht. Man filtriert ab, wäscht das Kristallisat mehrfach mit Diäthyläther nach, trocknet es: 10 g schmutzig-rotes Kristallisat, das mehrmals aus Methylenchlorid/Aceton/Diäthyläther umkristallisiert wird.

Ausbeute: 2,9 g rötliches Verfahrensprodukt vom Schmp. 198 - 214° C (sehr unscharf, was auf das Vorliegen eines Isomerengemisches von Anthrachinon-hydroxy-bis-(oxy-2,3-oxido-propan) zurückgeführt wird).

$^1$H-NMR-Spektrum ($DCCl_3$):

Charakteristisches Signalmuster für 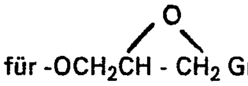 Gruppe:

δ = 2,8 ppm (AB-Teil eines ABX-Spektrums, $CH_2$-3)
δ = 3,45 ppm (m, CH-2)
δ = 4,3 ppm (AB-Teil eines ABX-Spektrums, $CH_2$-1)
MS: m/e = 368 (M+)

**Beispiel 17:**

Anthrachinon-dihydroxy-bis-(oxy-2,3-oxido-propan)

Eine Lösung von 6 g 1,4,5,8-Tetrahydroxy-anthrachinon in 150 ml Äthylmethylketon wird mit 6,15 g Kaliumcarbonat sowie 30 ml Epibromhydrin versetzt und solange unter gutem Rühren am Rückfluß gekocht, bis eine entnonmene Probe im MS-Spektrum einen deutlichen Molekulargewichtspeak bei m/e = 384 (M+) zeigt. Die höhermolekularen MS-Peaks bei > 384 sollten vergleichsweise klein ausfallen. Die Reaktionszeit beträgt hierbei in der Regel 3 bis 5 Tage.

Man läßt auf Zimmertemperatur abkühlen und filtriert die unlöslichen Anteile ab. Das Filtrat wird i. Vak. eingeengt und das resultierende Öl durch Anreiben mit ca. 20 ml Diäthyläther zur Kristallisation gebracht. Man filtriert ab, wäscht das Kristallisat mehrfach mit Diäthyläther nach, trocknet es: 11 g schmutzig-rotes Kristallisat, das mehrmals aus Methylenchlorid/Aceton/Diäthyläther umkristallisiert wird.

Ausbeute: 2,4 g rotes Verfahrensprodukt vom Schmp. 194 - 212°C (sehr unscharf, was auf das Vorliegen eines Isomerengemisches von Anthrachinon-dihydroxy-bis-(oxy-2,3-oxido-propan) zurückgeführt wird).

$^1$H-NMR-Spektrum ($DCCl_3$):

Charakteristisches Signalmuster für 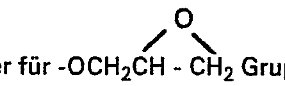 Gruppe:

$\delta$ = 2,8 ppm (AB-Teil eines ABX-Spektrums, $\underline{CH_2}$-3)
$\delta$ = 3,45 ppm (m $\underline{CH}$-2)
$\delta$ = 4,3 ppm (AB-Teil eines ABX-Spektrums, $\underline{CH_2}$-1)
MS-Spektrum: m/e = 348 (M+)

**Patentansprüche** für die Vertragsstaaten BE, CH, DE, FR, GB, IT, LI, LU, NL, SE

1. Verbindungen der Formel I

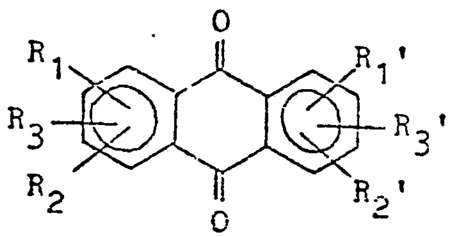

worin
$R_1$ und $R_1'$ gleich oder verschieden sind und Wasserstoffe, Hydroxy oder den Rest

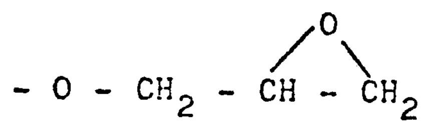

bedeuten,
$R_2$ und $R_2'$ gleich oder verschieden sind und Wasserstoff oder eine oder mehrere Hydroxy-, $C_1$-$C_4$-Alkyl-, $C_1$-$C_4$-Alkoxy-, $C_1$-$C_4$-Alkoxycarbonyl-, $C_1$-$C_4$-Alkanoyloxy, Nitrogruppen oder Halogenatome bedeuten und
$R_3$ und $R_3'$ verschieden sind und Wasserstoff oder den Rest

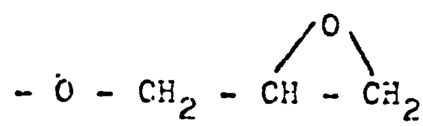

bedeuten.

2. 1-Hydroxy-anthrachinon-4-(oxy-2,3-oxido-propan).
3. Anthrachinon-1,4-bis-(oxy-2,3-oxido-propan).
4. Anthrachinon-2,6-bis-(oxy-2,3-oxido-propan).
5. Anthrachinon-1,8-bis-(oxy-2,3-oxido-propan).
6. Verbindung der Formel I gemäß Anspruch 1 zur Verwendung bei der Behandlung von malignen Tumorerkrankungen.
7. Verfahren zur Herstellung von Verbindungen der Formel I

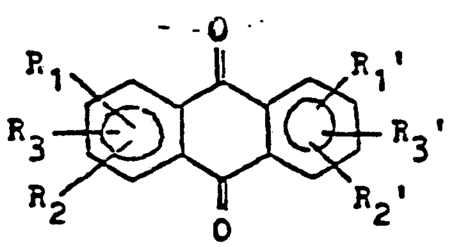

I

worin
$R_1$ und $R_1'$ gleich oder verschieden sind und Wasserstoff, Hydroxy oder den Rest

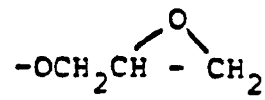

bedeuten,
$R_2$ und $R_2'$ gleich oder verschieden sind und Wasserstoff oder eine oder mehrere Hydroxy-, $C_1$-$C_4$-Alkyl-, $C_1$-$C_4$-Alkoxy-, $C_1$-$C_4$-Alkoxycarbonyl-, $C_1$-$C_4$-Alkanoyloxy-, Halogen- oder Nitrogruppen bedeuten, und
$R_3$ und $R_3'$ verschieden sind und Wasserstoff oder den Rest

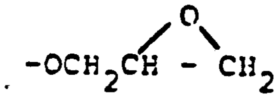

bedeuten, dadurch gekennzeichnet, daß man
a) eine Verbindung der Formel II

II 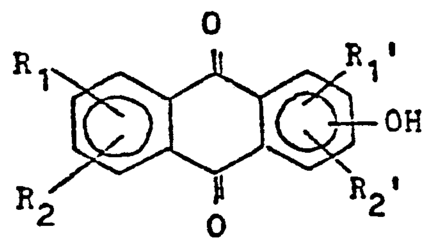 (Position 1,2,3,4,5, 6,7 oder 8)

worin
$R_1$ und $R_1'$ H und/oder OH bedeuten, und
$R_2$ und $R_2'$ die oben angegebene Bedeutung haben,
mit Epichlor- oder Epibromhydrin in Gegenwart von Basen zu einer Verbindung der Formel I umsetzt, oder
b) eine Verbindung der Formel III

III 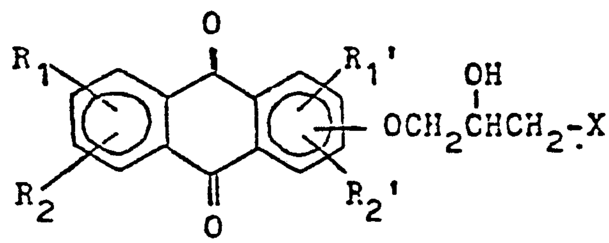

worin
$R_1$ und $R_1'$ H, OH oder $OCH_2\overset{OH}{C}HCH_2Hal$ bedeuten,
X ein Halogenatom bedeutet, und
$R_2$ und $R_2'$ die oben angegebene Bedeutung haben,
mit anorganischen oder organischen Basen zu einer Verbindung der Formel I umsetzt, oder
c) in einer Verbindung der Formel V

V 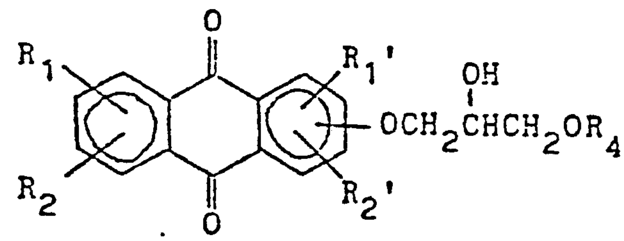

worin

$R_1$ und $R_1'$ H, OH oder den Rest $OCH_2\overset{OH}{C}HCH_2OR_4$ und
$R_4$ einen Alkyl- oder Arylsulfonsäurerest bedeuten und
$R_2$ und $R_2'$ sowie $R_4$ die oben angegebenen Bedeutungen haben,
gegebenenfalls nach nucleophilem Austausch der Alkyloder Arylsulfonsäureestergruppe gegen Halogen, durch Behandlung mit Basen Alkylsulfonsäure oder Arylsulfonsäure oder gegebenenfalls Halogenwasserstoff abspaltet, wodurch eine Verbindung der Formel I entsteht, oder

d) eine Verbindung der Formel X

X 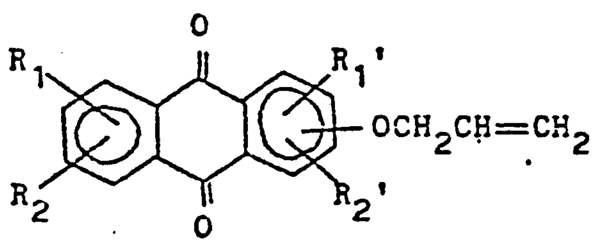

worin
$R_1$ und $R_1'$ H, OH oder $OCH_2CH{=}CH_2$ bedeuten und
$R_2$ und $R_2'$ die obigen Bedeutungen haben
mit organischen Alkyl- oder Arylpercarbonsäuren in inerten organischen Lösungsmitteln oder mit dem ($J_2 \cdot Ag_2O$)-Komplex zu den erfindungsgemäßen Verbindungen der Formel I umsetzt.

8. Arzneimittel, gekennzeichnet durch den Gehalt an einer Verbindung der Formel 1 gemäß Anspruch 1.

**Patentanspruch** für den Vertragsstaat AT

Verfahren zur Herstellung von Verbindungen der Formel I

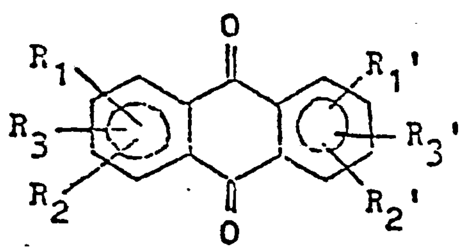 I

worin
$R_1$ und $R_1'$ gleich oder verschieden sind und Wasserstoff, Hydroxy oder den Rest

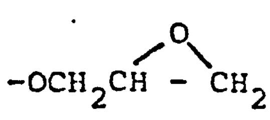

bedeuten,
$R_2$ und $R_2'$ gleich oder verschieden sind und Wasserstoff oder eine oder mehrere Hydroxy-, $C_1$-$C_4$-Alkyl-, $C_1$-$C_4$-Alkoxy-, $C_1$-$C_4$-Alkoxycarbonyl-, $C_1$-$C_4$-Alkanoyloxy-, Halogen- oder Nitrogruppen bedeuten, und

$R_1$ und $R_3'$ verschieden sind und Wasserstoff oder den Rest

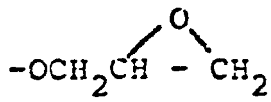

bedeuten, dadurch gekennzeichnet, daß man

a) eine Verbindung der Formel II

II

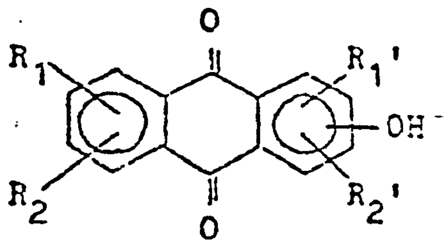

worin

$R_1$ und $R_1'$ H und/oder OH bedeuten, und

$R_2$ und $R_2'$ die oben angegebene Bedeutung haben,

mit Epichlor- oder Epibromhydrin in Gegenwart von Basen zu einer Verbindung der Formel 1 umsetzt, oder

b) eine Verbindung der Formel III

III

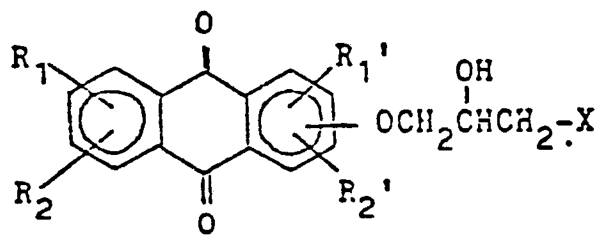

worin

$R_1$ und $R_1'$ H, OH oder $OCH_2\overset{OH}{C}HCH_2Hal$ bedeuten,

X ein Halogenatom bedeutet, und

$R_2$ und $R_2'$ die oben angegebene Bedeutung haben,

mit anorganischen oder organischen Basen zu einer Verbindung der Formel I umsetzt, oder

c) in einer Verbindung der Formel V

V

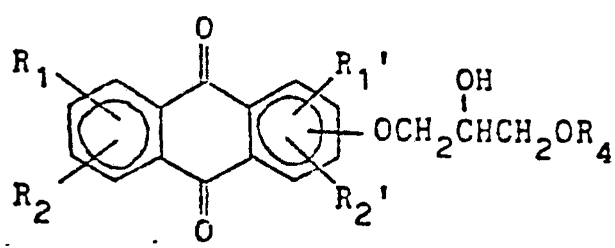

worin

$R_1$ und $R_1'$ H, OH oder den Rest $OCH_2\overset{OH}{C}HCH_2OR_4$ und

$R_4$ einen Alkyl- oder Arylsulfonsäurerest bedeuten und

$R_2$ und $R_2'$ sowie $R_4$ die eben angegebenen Bedeutungen haben,

gegebenenfalls nach nucleophilem Austausch der Alkyl- oder Arylsulfonsäureestergruppe gegen Halogen, durch Behandlung mit Basen Alkylsulfonsäure oder Arylsulfonsäure oder gegebenenfalls Halogenwasserstoff abspaltet, wodurch eine Verbindung der Formel I entsteht, oder

d) eine Verbindung der Formel X

X 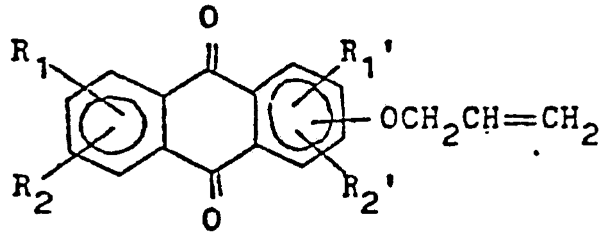

worin
$R_1$ und $R_1'$ H, OH oder $OCH_2CH=CH_2$ bedeuten und
$R_2$ und $R_2'$ die obigen Bedeutungen haben
mit organischen Alkyl- oder Arylpercarbonsäuren in inerten organischen Lösungsmitteln oder mit dem ($J_2 \cdot Ag_2O$)-Komplex zu den erfindungsgemäßen Verbindungen der Formel I umsetzt.

**Claims** for the Contracting states: BE, CH, DE, FR, GB, IT, LI, LU, NL, SE

1. A compound of the formula

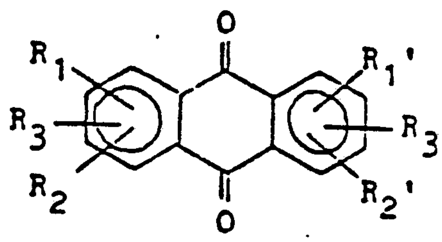

where
$R_1$ and $R_1'$ are identical or different and are hydrogen, hydroxyl or the radical

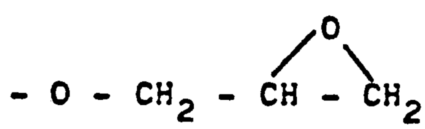

$R_2$ and $R_2'$ are identical or different and are hydrogen or one or more hydroxyl, $C_1$-$C_4$-alkyl, $C_1$-$C_4$-alkoxy, $C_1$-$C_4$-alkoxycarbonyl, $C_1$-$C_4$-alkanoyloxy, nitro groups or halogen atoms, and $R_3$ and $R_3'$ are different and are hydrogen or the radical

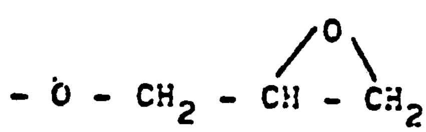

2. 1-Hydroxy-anthraquinone-4-(oxy-2,3-oxido-propane).
3. Anthraquinone-1,4-bis-(oxy-2,3-oxido-propane).
4. Anthraquinone-2,6-bis-(oxy-2,3-oxido-propane).
5. Anthraquinone-1,8-bis-(oxy-2,3-oxido-propane).
6. A compound of the formula I as claimed in claim 1 for use in the treatment of malignant tumoral diseases.
7. A process for the preparation of a compound of the formula I

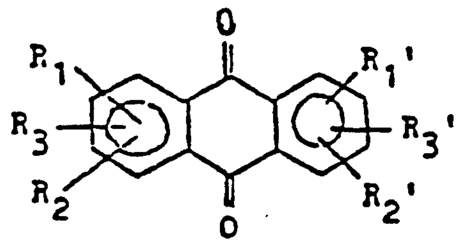

I

where

$R_1$ and $R_1'$ are identical or different and are hydrogen, hydroxyl or the radical

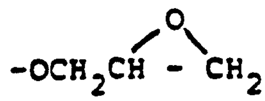

$R_2$ and $R_2'$ are identical or different and are hydrogen or one or more hydroxyl, $C_1$-$C_4$-alkyl, $C_1$-$C_4$-alkoxy, $C_1$-$C_4$-alkoxycarbonyl, $C_1$-$C_4$-alkanoyloxy, halogen or nitro groups, and

$R_3$ and $R_3'$ are different and are hydrogen or the radical

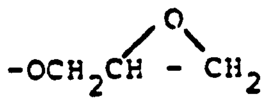

wherein

a) a compound of the formula II

II

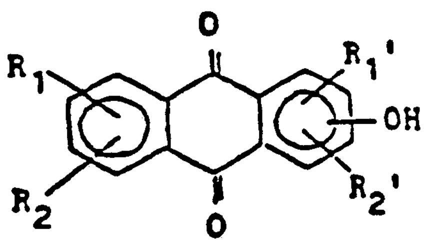

(Position 1,2,3,4,5, 6,7 or 8)

where

$R_1$ and $R_1''$ are H and/or OH and

$R_2$ and $R_2'$ have the above meaning,

is reacted with epichlorohydrin or epibromohydrin in the presence of a base to give a compound of the formula I or

b) a compound of the formula III

III

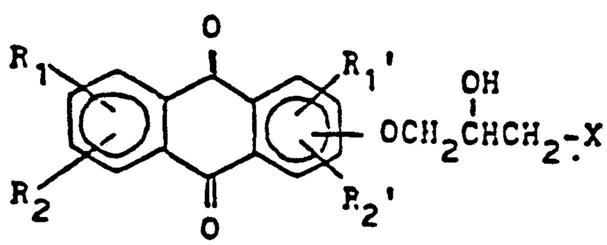

where

$R_1$ and $R_1'$ are H, OH or $OCH_2\overset{\overset{OH}{|}}{C}HCH_2Hal$,

X is halogen and

$R_2$ and $R_2'$ have the above meaning,

is reacted with an inorganic or organic base to give a compound of the formula I, or

c) in a compound of the formula V

V 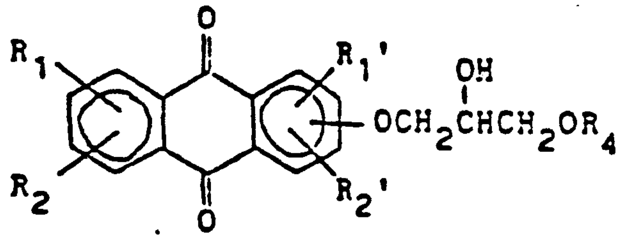

where

$R_1$ and $R_1''$ are H, OH or the radical $OCH_2\overset{OH}{\overset{|}{C}}HCH_2OR_4$ and
$R_4$ is an alkylsulfonic acid or arylsulfonic acid radical and
$R_2$ and $R_2'$ as well as $R_4$ have the above meanings, if appropriate after nucleophilic replacement of the alkylsulfonic acid or arylsulfonic acid ester group by halogen, the alkylsulfonic acid or arylsulfonic acid or, where appropriate, hydrogen halide is split off by treatment with a base, resulting in a compound of the formula I, or

d) a compound of the formula X.

X 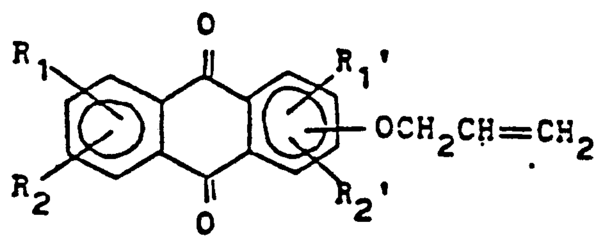

where
$R_1$ and $R_1''$ are H, OH or $OCH_2CH{=}CH_2$ and
$R_2$ and $R_2'$ have the above meanings
is reacted with an organic alkylpercarboxylic acid or arylpercarboxylic acid in an inert organic solvent, or with the $(I_2 \cdot Ag_2O)$ complex, to give a compound according to the invention, of the formula I.

8. A drug containing a compound of the formula I as claimed in claim 1.

**Claim** for the contracting state AT

A process for the preparation of a compound of the formula I

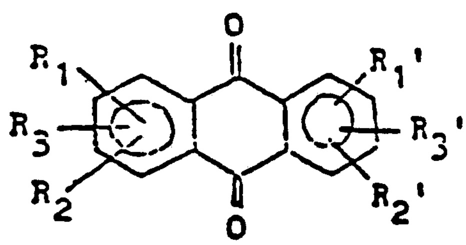 I

where
$R_1$ and $R_1'$ are identical or different and are hydrogen, hydroxyl or the radical

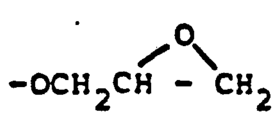

$R_2$ and $R_2'$ are identical or different and are hydrogen or one or more hydroxyl, $C_1$-$C_4$-alkyl, $C_1$-$C_4$-alkoxy, $C_1$-$C_4$-alkoxycarbonyl, $C_1$-$C_4$-alkanoyloxy, halogen or nitro groups, and
$R_3$ and $R_3'$ are different and are hydrogen or the radical

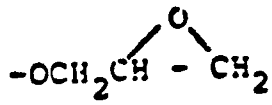

wherein
a) a compound of the formula II

II

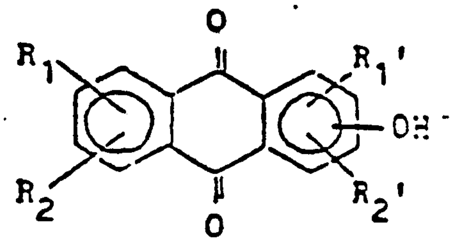

where $R_1$ and $R_1'$ are H and/or OH and $R_2$ and $R_2'$ have the above meaning, is reacted with epichlorohydrin or epibromohydrin in the presence of a base to give a compound of the formula I or
b) a compound of the formula III

III

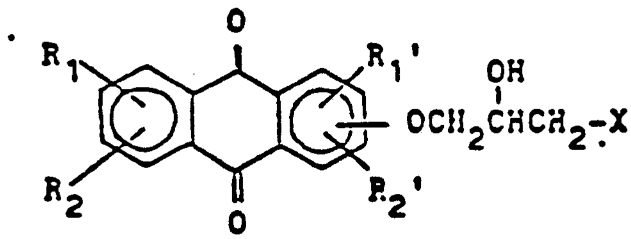

where

OH
|
$R_1$ and $R_1'$ are H, OH or $OCH_2CHCH_2Hal$, X is halogen and
$R_2$ and $R_2'$ have the above meaning,
is reacted with an inorganic or organic base to give a compound of the formula I, or
c) in a compound of the formula V

V

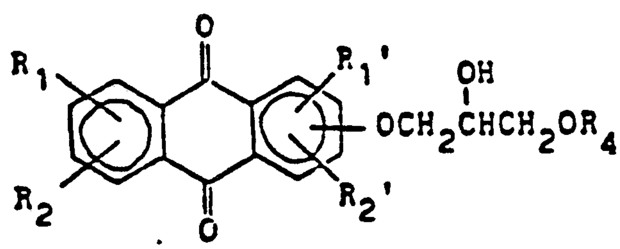

where
OH
|
$R_1$ and $R_1'$ are H, OH or the radical $OCH_2CHCH_2OR_4$ and
$R_4$ is an alkylsulfonic acid or arylsulfonic acid radical and
$R_2$ and $R_2'$ as well as $R_4$ have the above meanings,
if appropriate after nucleophilic replacement of the alkylsulfonic acid or arylsulfonic acid ester group by

halogen, the alkylsulfonic acid or arylsulfonic acid or, where appropriate, hydrogen halide is split off by treatment with a base, resulting in a compound of the formula I, or

d) a compound of the formula X

X

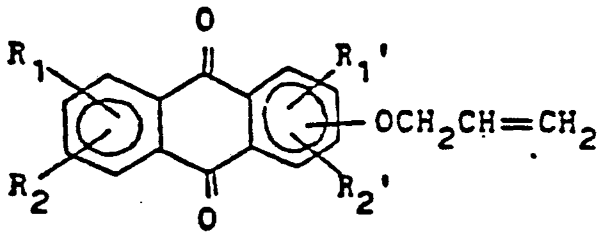

where

$R_1$ and $R_1'$ are H, OH or $OCH_2CH=CH_2$ and

$R_2$ and $R_2'$ have the above meanings

is reacted with an organic alkylpercarboxylic acid or arylpercarboxylic acid in an inert organic solvent, or with the ($I_2 \cdot Ag_2O$) complex, to give a compound according to the invention, of the formula I.

**Revendications** pour les Etats contractants: BE, CH, DE, FR, GB, IT, LI, LU, NL, SE

1. Composés de formule I

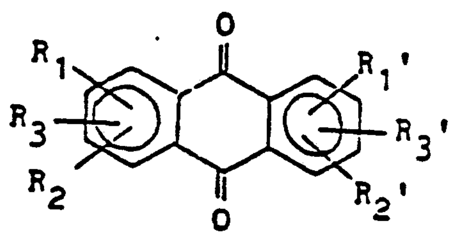

I

dans laquelle

$R_1$ et $R_1'$ sont identiques ou différents, et représentent un atome d'hydrogène, un groupe hydroxy ou le radical

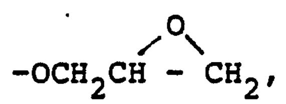

$R_2$ et $R_2'$ sont identiques ou différents, et représentent un atome d'hydrogène ou un ou plusieurs atomes d'halogène ou groupes hydroxy, alkyle en $C_1$-$C_4$, alcoxy en $C_1$-$C_4$, alcoxy ($C_1$-$C_4$)-carbonyle, alcanoyloxy en $C_1$-$C_4$ ou nitro, et

$R_3$ et $R_3'$ sont différents, et représentent un atome hydrogène ou le radical

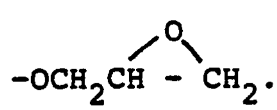

2. 1-hydroxy-anthraquinone-4-(oxy-2,3-oxydo-propane).
3. Anthraquinone-1,4-bis-(oxy-2,3-oxydo-propane).
4. Anthraquinone-2,6-bis-(oxy-2,3-oxydo-propane).
5. Anthraquinone-1,8-bis-(oxy-2,3-oxydo-propane).
6. Composé de formule I selon la revendication 1, pour utilisation dans le traitement d'affections à tumeurs malignes.
7. Procédé pour la préparation de composés de formule I

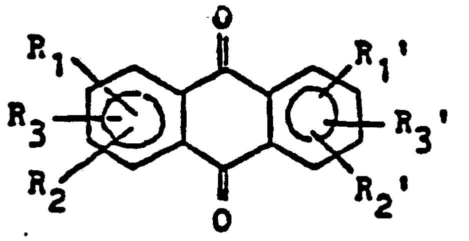

I

dans laquelle

$R_1$ et $R_1'$ sont identiques ou différents, et représentent un atome d'hydrogène, un groupe hydroxy ou le radical

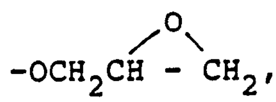

$R_2$ et $R_2'$ sont identiques ou différents, et représentent un atome d'hydrogène ou un ou plusieurs atomes d'halogène ou groupes hydroxy, alkyle en $C_1$-$C_4$, alcoxy en $C_1$-$C_4$, alcoxy($C_1$-$C_4$)-carbonyle, alcanoyloxy en $C_1$-$C_4$ ou nitro, et

$R_3$ et $R_3'$ sont différents, et représentent un atome d'hydrogène ou le radical

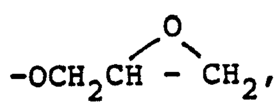

caractérisé en ce que

a) on fait réagir un composé de formule II

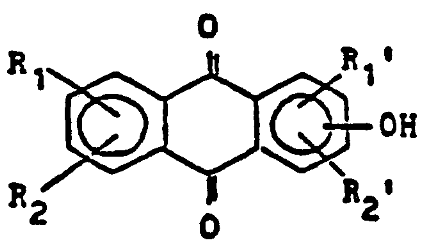

(position 1,2,3,4,5,6,7 ou 8 ) II

dans laquelle

$R_1$ et $R_1'$ représentent H et/ou OH, et

$R_2$ et $R_2'$ ont les significations données plus haut,

avec l'épichlorhydrine ou l'épibromhydrine en présence de bases, pour aboutir à un composé de formule I, ou

b) on fait réagir un composé de formule III

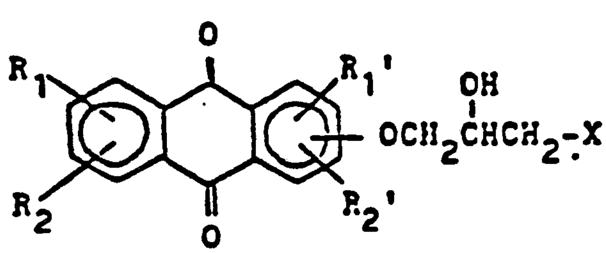

III

dans laquelle

OH

$R_1$ ét $R_1'$ représentent H, OH ou $OCH_2CHCH_2Hal$, X représente un atome d'halogène, et
$R_2$ et $R_2'$ ont les significations données plus haut,
avec des bases organiques ou minérales, pour aboutir à un composé de formule I, ou
c) dans un composé de formule V

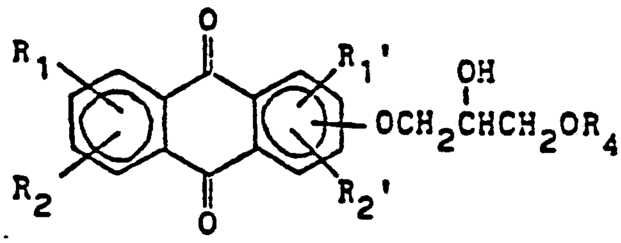

V

dans laquelle

OH

$R_1$ et $R_1'$ représentent H, OH ou le radical $OCH_2CHCH_2OR_4$, et
$R_4$ représente un reste alkyl- ou arylsulfonyle, et
$R_2$ et $R_2'$ ainsi que $R_4$ ont les significations données plus haut,
on sépare l'acide alkylsulfonique ou l'acide arylsulfonique, ou éventuellement l'hydracide halogéné, par traitement par des bases, éventuellement après échange nucléophile du groupe ester alkyl- ou arylsulfonique contre un halogène, ce par quoi il en résulte un composé de formule I, ou
d) on fait réagir un composé de formule X

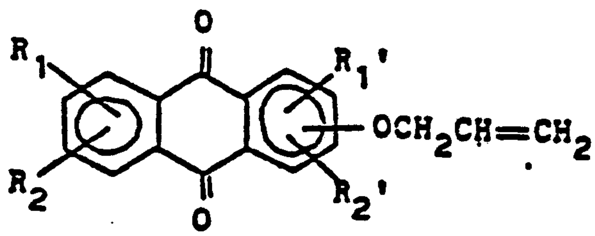

X

dans laquelle
$R_1$ et $R_1'$ représentent H, OH ou $OCH_2CH=CH_2$, et
$R_2$ et $R_2'$ ont les significations données plus haut, avec des acides organiques alkyl- ou arylpercarboxyliques, dans des solvants organiques inertes, ou avec le complexe ($I_2 \cdot Ag_2O$), pour aboutir aux composés de formule I selon l'invention.

8. Médicament caractérisé par une teneur en un composé de formule I selon la revendication 1.

**Revendication** pour l'Etat Contractant AT

Procédé pour la préparation de composés de formule I

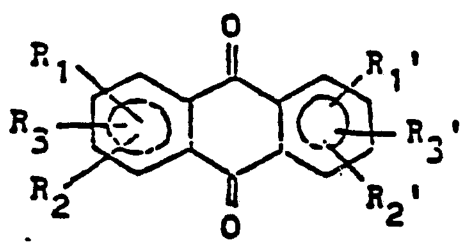

I

dans laquelle
$R_1$ et $R_1'$ sont identiques ou différents et représentent un atome d'hydrogène, un groupe hydroxy ou le radical

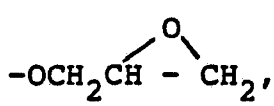

$R_2$ et $R_2'$ sont identiques ou différents, et représentent un atome d'hydrogène ou un ou plusieurs atomes d'halogène ou groupes hydroxy, alkyle en $C_1$-$C_4$, alcoxy en $C_1$-$C_4$, alcoxy($C_1$-$C_4$)-carbonyle, alcanoyloxy en $C_1$-$C_4$ ou nitro, et

$R_3$ et $R_3'$ sont différents, et représentent un atome d'hydrogène ou le radical

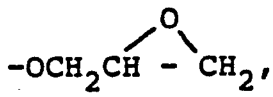

caractérisé en ce que

a) on fait réagir un composé de formule II

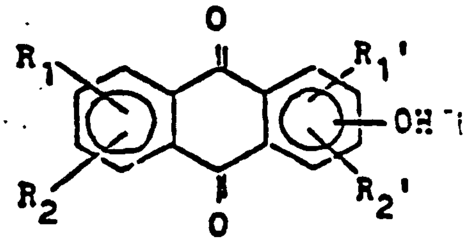

II

dans laquelle

$R_1$ et $R_1'$ représentent H et/ou OH et

$R_2$ et $R_2'$ ont les significations données plus haut,

avec l'épichlorhydrine ou l'épibromhydrine en présence de bases, pour aboutir à un composé de formule I, ou

b) on fait réagir un composé de formule III

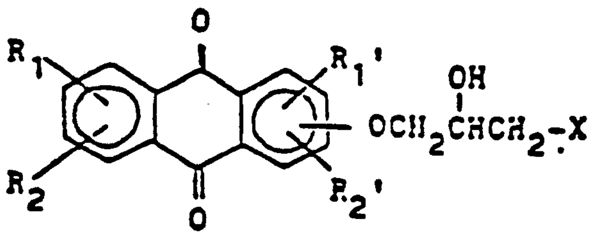

III

dans laquelle

$R_1$ et $R_1'$ représentent H, OH ou $OCH_2CH(OH)CH_2Hal$,

X représente un atome d'halogène, et

$R_2$ et $R_2'$ ont les significations données plus haut,

avec des bases organiques ou minérales, pour aboutir à un composé de formule I, ou

c) dans un composé de formule V

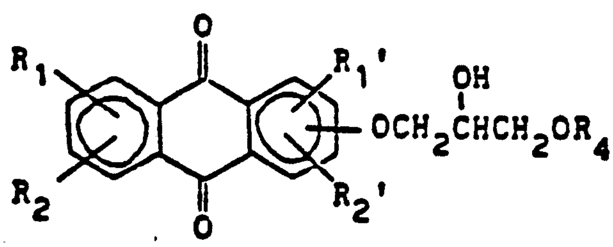

V

dans laquelle

$R_1$ et $R_1'$ représentent H, OH ou le radical $OCH_2CH(OH)CH_2OR_4$, et

$R_4$ représente un reste alkyl- ou arylsulfonyle, et

$R_2$ et $R_2'$ ainsi que $R_4$ ont les significations données plus haut,

on sépare l'acide alkylsulfonique ou l'acide arylsulfonique, ou éventuellement l'hydracide halogéné, par traitement par des bases, éventuellement après échange nucléophile du groupe ester alkyl- ou arylsulfonique contre un halogène, ce par quoi il en résulte un composé de formule I, ou

d) on fait réagir un composé de formule X

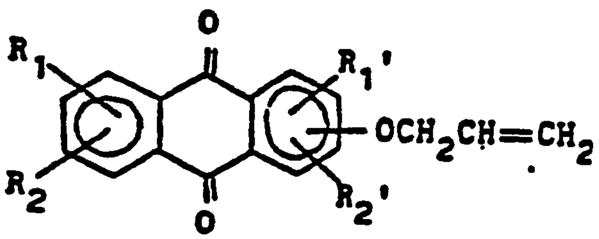

X

dans laquelle

$R_1$ et $R_1'$ représentent H, OH ou $OCH_2CH=CH_2$, et

$R_2$ et $R_2'$ ont les significations données plus haut,

avec des acides organiques alkyl- ou arylpercarboxyliques, dans des solvants organiques inertes, ou avec le complexe ($I_2 \cdot Ag_2O$), pour aboutir aux composés de formule I selon l'invention.